# DEMANDE DE BREVET EUROPEEN

(11) **EP 0 818 449 A1**
(43) Date de publication de la demande: **14.01.1998**
(21) Numéro de dépôt: 97401655.2
(22) Date de dépôt: 10.07.1997
(51) Int. Cl.: C07D 215/36, A61K 31/47, C07D 401/04, C07D 471/04

(54) **Dérivés de 2-(1H)-quinoléinone en tant qu'antagonistes d'acides aminés excitateurs**

(30) Priorité: 11.07.1996 FR 9608667
(71) Demandeur: ADIR ET COMPAGNIE, 92415 Courbevoie Cédex (FR)
(72) Inventeur: Cordi, Alex, 92150 Suresnes (FR); Desos, Patrice, 92400 Courbevoie (FR); Lepagnol, Jean, 28210 Chaudon (FR); Morain, Philippe, 92130 Issy les Moulineaux (FR); Lestage, Pierre, 78170 La celle Saint Cloud (FR)

(57) **Abrégé**

Composés de formule (I): 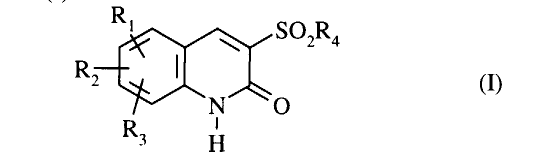 dans laquelle :
R₁, R₂, R₃, identiques ou différents, représentent un atome d'hydrogène, d'halogène, un groupement alkyle, un groupement nitro, cyano, aminosulfonyl, imidazolyl ou pyrrolyl, ou bien, lorsque deux d'entre eux sont situés sur des carbones adjacents, forment avec les atomes de carbone auxquels ils sont attachés, un cycle benzénique,
R₄ représente un groupement hydroxy, alkoxy, phénoxy ou amino,
leurs isomères ainsi que leurs sels d'addition à une base pharmaceutiquement acceptable.

## Description

La présente invention concerne de nouveaux dérivés de 2-(1H)-quinoléinone, leur procédé de préparation et les compositions pharmaceutiques qui les contiennent.

Quelques dérivés de 2-(1H)-quinoléinone ont été décrits dans la littérature. C'est le cas, par exemple, des composés décrits par C. ALABASTER et coll. (J. Med. Chem., 31, 2048-2056, 1988) qui sont des stimulants cardiaques ou de ceux décrits par F. BAHR et coll. (Pharmazie, 36, H.10, 1981).

Les composés décrits dans la présente invention, outre le fait qu'ils soient nouveaux, présentent des propriétés pharmacologiques particulièrement intéressantes : ce sont de puissants inhibiteurs des phénomènes liés à l'hyperactivation neuronale provoquée par les aminoacides excitateurs.

L'acide L-glutamique et l'acide L-aspartique ont la capacité d'activer les neurones du système nerveux central et de nombreux travaux ont démontré que ces aminoacides excitateurs (AAE) répondent aux critères définissant un neurotransmetteur. C'est pourquoi la modulation des événements neuronaux liés à ces AAE apparait être une cible intéressante pour le traitement des maladies neurologiques.

En effet, il a été prouvé que la libération exagérée des AAE et l'hyperstimulation de leurs récepteurs seraient une des causes de la dégénérescence neuronale que l'on observe dans l'épilepsie, la démence sénile ou les accidents vasculaires cérébraux. Actuellement, le nombre de maladies neurodégénératives dans lesquelles les AAE sont étroitement impliqués ne cesse de grandir (chorée de Huntington, schizophrénie, sclérose amyotrophique latérale) (Mc GEER E.G. et al., Nature, 263, 517-519, 1976 ; SIMON R. et al., Science, 226, 850-852, 1984).

De plus, s'il est certain que l'hyperactivation de la neurotransmission aux AAE exerce des effets neurotoxiques, l'activation normale de celle-ci facilite les performances mnésiques et cognitives (LYNCH G. & BAUDRY M., Science, 224, 1057-1063, 1984 ; ROTHMAN S.M. & OLNEY J.W., Trends in Neuro Sci., 10, 299-302, 1987). Du point de vue pharmacologique et thérapeutique, il convient donc de ne s'opposer qu'aux stimulations pathologiques tout en respectant le niveau d'activation physiologique.

Les récepteurs aux AAE à localisation post- et présynaptique ont été classés en 4 groupes en fonction de l'affinité et des effets électrophysiologiques et/ou neurochimiques de ligands spécifiques :
. récepteur NMDA (N-méthyl-D-aspartate) associé à un canal ionique perméable aux cations mono- et divalents (dont le calcium) mais qui est bloqué par le magnésium. L'accumulation du calcium et du zinc dans la cellule serait une des causes de la mort neuronale. L'ouverture du canal NMDA est régulée par plusieurs sites associés au récepteur et en particulier est favorisée par la glycine dont l'effet est strychnine-insensible. Ce site glycine constitue l'une des cibles importantes pour moduler l'activation du récepteur NMDA.
. récepteur AMPA (acide α-amino-3-hydroxy-5-méthyl-4-isoxazole-propionique) associé à un canal ionique perméable aux cations monovalents dont le sodium. L'activation de ce canal entrainerait une dépolarisation membranaire.
. récepteur kainate dont les caractéristiques ioniques sont proches du récepteur AMPA mais qui s'en diffère par les niveaux de conductance et de désensibilisation. Toutefois, de nombreuses études tendent à prouver que le récepteur AMPA et le récepteur kainate ont d'étroites analogies structurales et fonctionnelles et constituent une même famille réceptorielle (KEINANEN K. et al., Science, 249, 556-560, 1990).
. récepteur ACPD (acide-trans-1-aminocyclopentane-1,3-dicarboxylique) appelé récepteur métabotropique car non couplé à un canal ionique.

L'activation des récepteurs ionotropiques par les AAE ouvre les canaux ioniques et notamment permet l'entrée de sodium qui dépolarise la cellule. Cette première phase qui implique le récepteur AMPA, conduit alors à la desinhibition puis à l'hyperactivation du récepteur NMDA et à l'accumulation massive de calcium (BLAKE J.F. et al., Neurosci. Letters, 89, 182-186, 1988 ; BASHIR Z.I. et al., Nature, 349, 156-158, 1991).

Les composés de la présente invention ou les produits de leur hydrolyse métabolique (pro-drogues) visent donc de manière originale à s'opposer aux effets excitateurs et toxiques des AAE en bloquant l'activation initiale du récepteur à l'AMPA/kainate.

Les composés de la présente invention sont donc utiles en tant qu'inhibiteurs des phénomènes pathologiques notamment neurotoxiques liés à l'hyperactivation des voies de neurotransmission aux aminoacides excitateurs. Ils se singularisent vis-à-vis des produits décrits par la demande de brevet européen EP 542609 en ce qu'ils sont particulièrement solubles dans l'eau, dépourvus de toute coloration et qu'ils disposent d'une bonne biodisponibilité cérébrale après administration systémique.

Ils sont donc des agents thérapeutiques potentiels pour le traitement des maladies neurologiques et psychiques impliquant ces aminoacides : maladies dégénératives aigües ou chroniques, telles que l'accident vasculaire cérébral, le traumatisme cérébral ou spinal, l'épilepsie, les maladies neurodégénératives chroniques telles que la maladie d'Alzheimer, la schizophrénie, la sclérose amyotrophique latérale ou la chorée de Huntington.

Plus spécifiquement, la présente invention concerne les composés de formule (I): 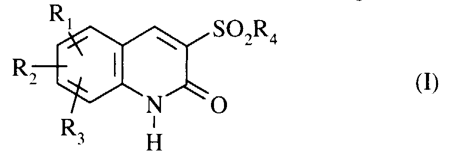 dans laquelle :
- R₁, R₂, R₃,: identiques ou différents, représentent un atome d'hydrogène, d'halogène, un groupement alkyle (C₁-C₆) linéaire ou ramifié (substitué ou non par un ou plusieurs atomes d'halogène), un groupement nitro, cyano, aminosulfonyl, imidazolyl (substitué ou non par un ou plusieurs groupements alkyle (C₁-C₆) linéaire ou ramifié ou aminoalkyle (C₁-C₆) linéaire ou ramifié) ou pyrrolyl (substitué ou non par un ou plusieurs groupements alkyle (C₁-C₆) linéaire ou ramifié ou aminoalkyle (C₁-C₆) linéaire ou ramifié),
ou bien, lorsque deux d'entre eux sont situés sur des carbones adjacents, forment avec les atomes de carbone auxquels ils sont attachés, un cycle benzénique (substitué ou non par un ou plusieurs atomes d'halogène, groupements alkyle (C₁-C₆) linéaire ou ramifié, alkoxy (C₁-C₆) linéaire ou ramifié ou trihalogénométhyle) ou un cycle cycloalkyle (C₃-C₇) dans lequel un groupement -CH₂- est éventuellement remplacé par un groupement -NR- (dans lequel R représente un atome d'hydrogène, un groupement alkyle (C₁-C₆) linéaire ou ramifié ou un groupement benzyle),
- R₄: représente un groupement hydroxy, alkoxy (C₁-C₆) linéaire ou ramifié, phénoxy (substitué ou non par un ou plusieurs atomes d'halogène, groupements alkyle (C₁-C₆) linéaire ou ramifié, alkoxy (C₁-C₆) linéaire ou ramifié ou trihalogénométhyle) ou amino (substitué ou non par un ou deux groupements alkyle (C₁-C₆) linéaire ou ramifié),
leurs isomères ainsi que leurs sels d'addition à une base pharmaceutiquement acceptable.

Parmi les bases pharmaceutiquement acceptables, on peut citer à titre non limitatif l'hydroxyde de sodium, l'hydroxyde de potassium, la tertbutylamine, la diéthylamine, l'éthylènediamine...

L'invention s'étend également au procédé de préparation des composés de formule (I) caractérisé en ce que l'on utilise comme produit de départ un composé de formule (II) : 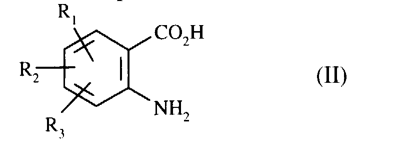 dans laquelle R₁, R₂ et R₃ ont la même signification que dans la formule (I),
que l'on réduit selon la nature des substituants R₂, R₃ et R₄,
soit en milieu aprotique en présence d'hydrure de lithium aluminium, d'hydrure d'aluminium, de diborane ou des complexes de borane,
soit en milieu protique acide lorsque l'on utilise le cyanoborohydrure de sodium, pour conduire à un alcool de formule (III) : 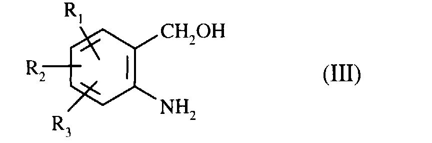 dans laquelle R₁, R₂ et R₃ ont la même signification que dans la formule (I),
que l'on oxyde à l'aide d'un oxyde métallique dans un solvant inerte, d'un hydrohalogénate de métal alcalin dans un solvant protique ou d'un chlorure d'acide dans le diméthylsulfoxyde, pour conduire à l'aldéhyde de formule (IV) : 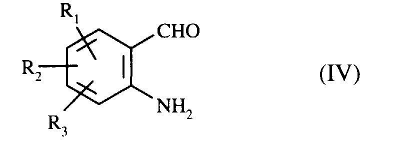 dans laquelle R₁, R₂ et R₃ ont la même signification que dans la formule (I),
qui est :
* soit condensé, avec un malonate d'alkyle de formule (V), en milieu protique, en présence d'un alcoolate de métal alcalin, d'une amine tertiaire ou d'un hydroxyde de métal alcalin selon le procédé décrit par A. Cordi et coll. (Biorg. Med. Chem., 1995, 2, 129-141) : 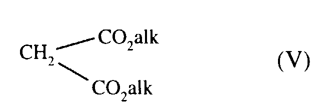 dans laquelle alk représente un groupement alkyle (C₁-C₆) linéaire ou ramifié, pour conduire à un ester de formule (VI) : 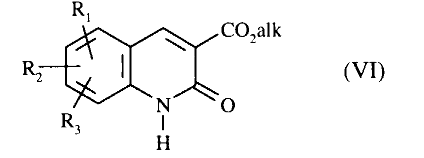 dans laquelle R₁, R₂, R₃ et alk ont la même signification que précédemment,
   que l'on transforme, en acide correspondant de formule (VII) : 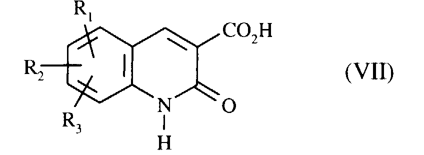 dans laquelle R₁, R₂ et R₃ ont la même signification que dans la formule (I),
   que l'on met en réaction en présence de brome dans de la pyridine, pour conduire au composé de formule (VIII) : 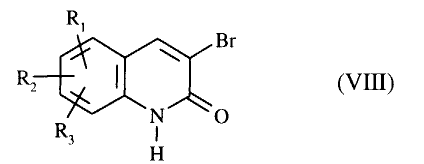 dans laquelle R₁, R₂ et R₃ ont la même signification que dans la formule (I),
   que l'on fait réagir avec un composé de formule (IX) selon le procédé décrit par P. Desos et coll. (J. Med. Chem., 1996, 39, 197-206) :

   R'SH (IX)

   dans laquelle R' représente un groupement alkyle (C₁-C₆) linéaire ou ramifié ou -CH₂CO₂alk (dans lequel alk a la même signification que précédemment),
   pour conduire au composé de formule (X) : 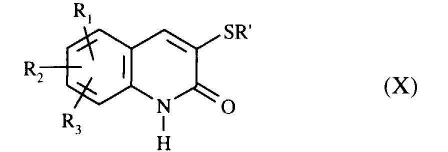 dans lequel R₁, R₂, R₃ et R' ont la même signification que dans la formule (I),
   qui subit une oxydation en présence d'acide nitrique dans l'acide sulfurique concentré, pour conduire à l'acide sulfonique de formule (I) qui peut être éventuellement transformé en ester correspondant,
* soit condensé, avec un composé de formule (XI), en milieu protique, en présence d'un alcoolate de métal alcalin, d'une amine tertiaire ou d'un hydroxyde de métal alcalin : 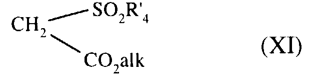 dans laquelle R'4 représente un groupement alkoxy (C₁-C₆) linéaire ou ramifié ou phénoxy éventuellement substitué, que l'on transforme :
   après traitement éventuel en milieu acide, en composé de formule (Ia), cas particulier des composés de formule (I) : 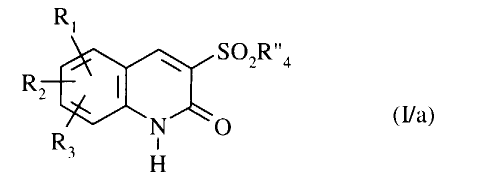 dans laquelle R₁, R₂ et R₃ sont tels que définis précédemment et R"₄ représente un groupement hydroxy, alkoxy (C₁-C₆) linéaire ou ramifié ou phénoxy éventuellement substitué,
   composé de formule (I/a), que l'on transforme éventuellement lorsque R"₄ représente un groupement hydroxy, en chlorure du sulfonyle correspondant, puis par action de l'amoniac ou d'une amine, en composé de formule (I/b), cas particulier des composés de formule (I) : 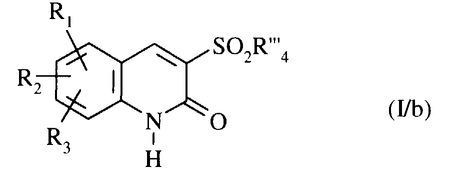 dans laquelle R₁, R₂, R₃ ont la même signification que dans la formule (I) et R'''₄ représente un groupement amino éventuellement substitué,
   composé de formule (I/a) ou (I/b),
   - qui, lorsque R₁ et/ou R₂ et/ou R₃ représentent un atome d'hydrogène, peut subir des substitutions électrophiles, selon des techniques classiques de substitution des noyaux aromatiques conduisant à un composé de formule (I) mono, di ou trisubstitué sur le noyau benzène de la quinoléinone,
   - qui, lorsque R₁ et/ou R₂ et/ou R₃ représentent un groupement nitro, peut subir une hydrogénation pour conduire au dérivé amino correspondant, qui, lui-même, peut être, si on le souhaite, transformé en dérivé cyano correspondant,
   - qui peut être, le cas échéant, purifié selon une technique classique de purification,
   - dont on sépare, le cas échéant, les isomères selon une technique classique de séparation,
   - que l'on transforme, si on le souhaite, en ses sels d'addition à un acide ou à une base pharmaceutiquement acceptable.

Les composés de formule (I) possèdent des propriétés pharmacologiques très intéressantes puisqu'ils s'opposent à l'activation des récepteurs glutamatergiques de type AMPA. Leur efficacité a pu être étudiée grâce aux techniques d'électrophysiologie *in vitro* et notamment grâce à la mesure des courants induits par les agonistes glutamatergiques dans l'oocyte de Xenopus.

Des oocytes de Xenopus sont injectés de 50 ng d'ARNm poly (A⁺) isolés de cortex cérébral de Rat et incubés 2 à 3 jours à 18°C pour en permettre l'expression protéique. Les courants entrants induits par les AAE sont mesurés dans un mileu de composition : NaCl (82,5 mM), KCl (2,5 mM), CaCl2 (1 mM), MgCl₂ (1 mM), NaH₂PO₄ (1 mM), HEPES (5 mM), pH 7,4 par la méthode du Voltage-clamp à 2 électrodes (potentiel = - 60 mV). Pour la mesure des courants induits par le NMDA et la glycine, MgCl₂ est absent du milieu et le CaCl₂ est amené à la concentration de 2 mM. Les agonistes AAE induisant les courants sont utilisés aux concentrations suivantes : kainate : 100 µM ; AMPA : 30 µM ; glycine/NMDA : 3/30 µM. Le produit étudié est appliqué 30 secondes avant et durant l'application de l'agoniste.

Dans ces conditions, les dérivés de la présente invention sont capables d'inhiber l'activation fonctionnelle du courant induite par l'AMPA. De plus, leur intérêt tient au fait qu'ils exercent cette inhibition de manière sélective et préférentielle. Leur interaction avec le récepteur NMDA n'est observée qu'à des concentrations supérieures, ce qui les rend dépourvus de tous les effets secondaires décrits pour les antagonistes NMDA (effets psychotomimétiques, amnésiants et neurotoxiques).

Ainsi, le composé de l'exemple 2 est 40 fois plus sélectif pour le récepteur AMPA, et cette inhibition est observée à faible concentration :
AMPA : IC50 = 0,2 µM
NMDA : IC50 = 8 µM.

La présente invention a également pour objet les compositions pharmaceutiques renfermant comme principe actif au moins un composé de formule (I), seul ou en combinaison avec un ou plusieurs excipients ou véhicules inertes non toxiques.

Parmi les compositions pharmaceutiques selon l'invention on pourra citer plus particulièrement celles qui conviennent pour l'administration orale, parentérale, nasale, les comprimés simples ou dragéifiés, les comprimés sublinguaux, les gélules, les tablettes, les suppositoires, les crèmes, pommades, gels dermiques, etc...

La posologie utile varie selon l'âge et le poids du patient, la nature et la sévérité de l'affection ainsi que la voie d'administration. Celle-ci peut être orale, nasale, rectale ou parentérale. D'une manière générale, la posologie unitaire s'échelonne entre 1 et 1000 mg pour un traitement en 1 à 3 prises par 24 heures.

Les exemples suivants illustrent l'invention et ne la limitent en aucune façon. Les produits de départ utilisés sont des produits connus ou préparés selon des modes opératoires connus.

### Exemple 1 : Acide 7-nitro-2-(1H)-quinoléinone-3-sulfonique

Dans 1 ml d'acide sulfurique 96 %, on dissout 1,00 g (3,24 mmol) de 3-[(carbéthoxyméthyl) thio]-7-nitro-2-*(1H)*-quinoléinone (préparé selon le procédé décrit par P. Desos et coll. (J. Med. Chem., **1996,** 39, 197-206) et on refroidit cette solution dans un bain de glace. On ajoute goutte à goutte 1 ml d'acide nitrique 86 %. A la fin de l'addition, on laisse le milieu réactionnel revenir à température ambiante et on poursuit l'agitation pendant 4 heures (précipitation dans le milieu réactionnel). Le milieu réactionnel est à nouveau refroidi à 5-10°C et on y ajoute un peu de glace. Après 10 minutes d'agitation, le précipité est filtré, rincé avec un peu d'eau puis d'éther. Le solide est séché sous vide puis est solubilisé dans l'isopropanol au reflux. On filtre à chaud quelques insolubles marrons et on dilue le filtrat avec de l'éther jusqu'à apparition d'un léger trouble. Le produit cristallise à température ambiante et est récupéré par filtration.
*Point de fusion :* > *300° C*

| *Microanalyse élémentaire :* | | | | |
|---|---|---|---|---|
| | *C%* | *H%* | *N%* | *S%* |
| *calculé* | *40,00* | *2,24* | *10,37* | *11,87* |
| *trouvé* | *39,67* | *2,29* | *10,20* | *12,28* |

### Exemple 2 : Acide 6,7-dinitro-2-(1H)-quinoléinone-3-sulfonique

Le composé de l'exemple 1 (790 mg, 2,79 mmol) est dissous dans 1 ml d'acide sulfurique 96 % et on ajoute goutte à goutte 1 ml d'acide nitrique 86 %. Le milieu réactionnel est chauffé à 120°C pendant 5 heures. On laisse revenir à température ambiante et on ajoute un peu de glace (le produit est soluble dans l'eau). Le précipité est filtré et rincé avec un minimum d'eau froide puis avec de l'acétonitrile. Le solide est ensuite agité 12 heures dans de l'acétonitrile et est récupéré par filtration.
*Point de fusion : > 300° C*

| *Microanalyse élémentaire :* | | | | |
|---|---|---|---|---|
| | *C%* | *H%* | *N%* | *S%* |
| *calculé* | *34,29* | *1,60* | *13,33* | *10,17* |
| *trouvé* | *34,06* | *1,76* | *12,86* | *10,86* |

### Exemple 3 : Acide 6,7-dichloro-2-(1H)-quinoléinone-3-sulfonique, sel de sodium

Une suspension contenant 2,00 g (10,5 mmol) de 4,5-dichloro-2-aminobenzaldéhyde (préparé selon le procédé décrit par A. Cordi et coll., Bioorg. Med. Chem., **1995**, *2*, 129-141), 4,12 g (21 mmol) d'ester éthylique de l'acide éthoxysulfonyl-acétique (préparé selon le procédé décrit par M. Bordeau et coll., Bull. Soc. Chim. Fr., **1986,** *3*, 413-417 et J.I. Trujillo et coll., Tetrahedron Lett., **1993,** *46*, 7355-7358), 4 ml (21 mmol) de méthanolate de sodium 5,25 M dans le méthanol et 25 ml de méthanol anhydre, est agitée 72 heures à température ambiante. Le méthanol est évaporé sous vide et le milieu réactionnel est neutralisé par addition goutte à goutte d'acide chlorhydrique 1N. Le précipité est filtré, repris dans 10 ml de diméthylformamide à chaud. On laisse revenir à température ambiante et on filtre le solide blanc.
*Point de fusion : > 300° C*

| *Microanalyse élémentaire :* | | | | | |
|---|---|---|---|---|---|
| | *C%* | *H%* | *N%* | *Cl%* | *S%* |
| *calculé* | *34,20* | *1,28* | *4,43* | *22,43* | *10,14* |
| *trouvé* | *33,83* | *1,37* | *4,44* | *21,88* | *9,91* |

Les exemples suivants ont été préparés selon le procédé décrit dans l'exemple 1 à partir des produits de départ correspondants :

### Exemple 4: Acide 6-(1H-imidazol-1-yl)-7-trifluorométhyl-2-(1H)-quinoléinone-3-sulfonique

### Exemple 5: Acide 6-(1H-imidazol-1-yl)-7-nitro-2-(1H)-quinoléinone-3-sulfonique

### Exemple 6: Acide 7-chloro-6-(1H-imidazol-1-yl)-2-(1H)-quinoléinone-3-sulfonique

### Exemple 7: Acide 6-(pyrrol-1-yl)-7-trifluorométhyl-2-(1H)-quinoléinone-3-sulfonique

### Exemple 8: Acide 7-nitro-6-(pyrrol-1-yl)-2-(1H)-quinoléinone-3-sulfonique

### Exemple 9: Acide 7-chloro-6-(pyrrol-1-yl)-2-(1H)-quinoléinone-3-sulfonique

### Exemple 10: Acide 7-cyano-6-(pyrrol-1-yl)-2-(1H)-quinoléinone-3-sulfonique

### Exemple 11: Acide 2-méthyl-5-chloro-8-oxo-1,2,3,4,7,8-hexahydro-[2,7]phénanthroline -9-sulfonique

### Exemple 12: Acide 2-méthyl-5-nitro-8-oxo-1,2,3,4,7,8-hexahydro-[2,7]phénanthroline -9-sulfonique

### Exemple 13: Acide 2-méthyl-5-trifluorométhyl-8-oxo-1,2,3,4,7,8-hexahydro-[2,7]phénanthroline-9-sulfonique

### Exemple 14: Acide 7-méthyl-2-oxo-2,6,7,8-tétrahydro-1H-pyrrolo[3,4-g]quinoline-3-sulfonique

### Exemple 15: Acide 2-méthyl-4-chloro-7-oxo-2,3,6,7-tétrahydro-1H-pyrrolo[3,4-f] quinoline-8-sulfonique

### Exemple 16: Acide 2-méthyl-4-nitro-7-oxo-2,3,6,7-tétrahydro-1H-pyrrolo[3,4-f] quinoline-8-sulfonique

### Exemple 17: Acide 2-méthyl-4-trifluorométhyl-7-oxo-2,3,6,7-tétrahydro-1H-pyrrolo[3,4-f]quinoline-8-sulfonique

### Etude pharmacologique des dérivés de l'invention

### Exemple 18: Activité anti-convulsivante in vivo

La libération excessive de glutamate est très largement impliquée dans le déclenchement des crises convulsives chez l'homme. Expérimentalement, le même phénomène peut être reproduit par injection d'agonistes glutamatergiques chez le Rat ou chez la Souris. De même, chez la Souris DBA2, des crises convulsives glutamate-dépendantes peuvent être déclenchées en soumettant les animaux à une stimulation sonore de haute fréquence (14 kHz, 103 dB). Les effets anti-convulsions sont alors objectivés par sa capacité à prévenir l'apparition des convulsions et la mort qui s'ensuit.

Administrés par voie i.p. 30 minutes avant d'imposer la stimulation sonore, les composés de l'invention exercent des effets anti-convulsivants et ces effets sont proportionnels aux doses administrés.

Ainsi, le composé de l'exemple 1 protège puissamment les animaux (ED50 = 26,9 mg/kg) et à la dose de 50 mg/kg, la mortalité est inhibée de plus de 80 % chez les animaux traités.

### Exemple 19: Activité anti-neurodégénérative in vivo

L'occlusion de l'artère sylvienne (ou artère cérébrale moyenne MCA) est à l'origine d'environ 70 % des cas d'accidents vasculaires cérébraux chez l'homme. Ce type d'ischémie cérébrale peut être reproduit très fidèlement chez le Rat par ligature intracérébrale de la MCA. Cette occlusion définitive entraîne en 24 heures la formation d'un infarctus striatocortical de localisation identique à celle observée en situation clinique. L'infarctus est parfaitement délimité donc mesurable histologiquement. Cet infarctus est de type oedémateux. Il se transforme dans les 3 jours suivants en infarctus nécrotique définitif. La libération excessive de glutamate est un élément pathologique majeur pour la genèse de l'infarctus cérébral. Un composé neuroprotecteur anti-ischémique dois s'opposer à la formation de cet infarctus à 24 heures et idéalement à sa maturation nécrotique à 96 heures.

Les composés de l'invention sont des composés à effet anti-ischémique puissant, même lors de traitement retardé post-ischémique (traitement par voie i.v. 1 heure après l'occlusion artérielle). Ainsi, à la dose de 20 mg/kg i.v., les composés des exemples 1 et 2 diminuent significativement le volume de l'infarctus oedemateux cortical de 45 et 27 % respectivement. Cette activité est majeure puisqu'ils s'opposent également à la maturation nécrotique. Ainsi, lorsqu'un traitement en relai durant 3 jours (10 mg/kg i.p. 2 fois par jour) est effectué après l'injection i.v. initiale (20 mg/kg), le composé de l'exemple 1 diminue de 44 % le volume de l'infarctus terminal.

### Exemple 20: Composition pharmaceutique

Formule de préparation pour 1000 comprimés dosés à 10 mg

| | |
|---|---|
| Composé de l'exemple 1 | 10 g |
| Hydroxypropylcellulose | 2 g |
| Amidon de blé | 10 g |
| Lactose | 100 g |
| Stéarate de magnésium | 3 g |
| Talc | 3 g |

## Revendications

1. Composés de formule (I) : 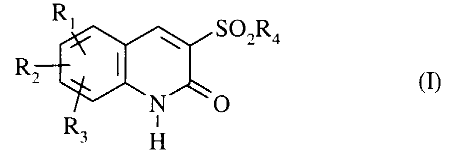 dans laquelle :
R₁, R₂, R₃, identiques ou différents, représentent un atome d'hydrogène, d'halogène, un groupement alkyle (C₁-C₆) linéaire ou ramifié (substitué ou non par un ou plusieurs atomes d'halogène), un groupement nitro, cyano, aminosulfonyl, imidazolyl (substitué ou non par un ou plusieurs groupements alkyle (C₁-C₆) linéaire ou ramifié ou aminoalkyle (C₁-C₆) linéaire ou ramifié) ou pyrrolyl (substitué ou non par un ou plusieurs groupements alkyle (C₁-C₆) linéaire ou ramifié ou aminoalkyle (C₁-C₆) linéaire ou ramifié),
ou bien, lorsque deux d'entre eux sont situés sur des carbones adjacents, forment avec les atomes de carbone auxquels ils sont attachés, un cycle benzénique (substitué ou non par un ou plusieurs atomes d'halogène, groupements alkyle (C₁-C₆) linéaire ou ramifié, alkoxy (C₁-C₆) linéaire ou ramifié ou trihalogénométhyle) ou un cycle cycloalkyle (C₃-C₇) dans lequel un groupement -CH2- est éventuellement remplacé par un groupement -NR- (dans lequel R représente un atome d'hydrogène, un groupement alkyle (C₁-C₆) linéaire ou ramifié ou un groupement benzyle),
R₄ représente un groupement hydroxy, alkoxy (C₁-C₆) linéaire ou ramifié, phénoxy (substitué ou non par un ou plusieurs atomes d'halogène, groupements alkyle (C₁-C₆) linéaire ou ramifié, alkoxy (C₁-C₆) linéaire ou ramifié ou trihalogénométhyle) ou amino (substitué ou non par un ou deux groupements alkyle (C₁-C₆) linéaire ou ramifié),
leurs isomères ainsi que leurs sels d'addition à une base pharmaceutiquement acceptable.

2. Composés de formule (I) selon la revendication 1 tels que l'un au moins des groupements R₁, R₂ ou R₃ représente un groupement nitro.

3. Composés de formule (I) selon la revendication 1 tels que R4 représente un groupement hydroxy.

4. Composé de formule (I) selon la revendication 1 qui est l'acide 7-nitro-2-*(1H)*-quinoléinone-3-sulfonique.

5. Composé de formule (I) selon la revendication 1 qui est l'acide 6,7-dinitro-2-(1H)-quinoléinone-3-sulfonique.

6. Composé de formule (I) selon la revendication 1 qui est l'acide 6,7-dichloro-2-(*1H*)-quinoléinone-3-sulfonique, sel de sodium.

7. Procédé de préparation des composés de formule (I) caractérisé en ce que l'on utilise comme produit de départ un composé de formule (II) : 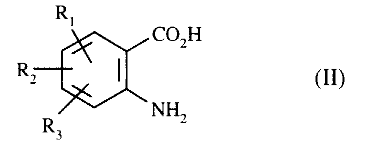 dans laquelle R₁, R₂ et R₃ ont la même signification que dans la formule (I),
que l'on réduit selon la nature des substituants R₂, R₃ et R₄,
soit en milieu aprotique en présence d'hydrure de lithium aluminium, d'hydrure d'aluminium, de diborane ou des complexes de borane,
soit en milieu protique acide lorsque l'on utilise le cyanoborohydrure de sodium, pour conduire à un alcool de formule (III) : 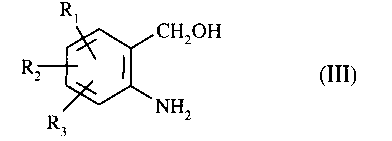 dans laquelle R₁, R₂ et R₃ ont la même signification que dans la formule (I),
que l'on oxyde à l'aide d'un oxyde métallique dans un solvant inerte, d'un hydrohalogénate de métal alcalin dans un solvant protique ou d'un chlorure d'acide dans le diméthylsulfoxyde, pour conduire à l'aldéhyde de formule (IV) : 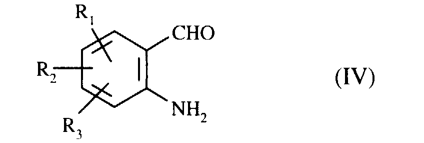 dans laquelle R₁, R₂ et R₃ ont la même signification que dans la formule (I),
qui est :
* soit condensé, avec un malonate d'alkyle de formule (V), en milieu protique, en présence d'un alcoolate de métal alcalin, d'une amine tertiaire ou d'un hydroxyde de métal alcalin : 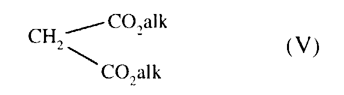 dans laquelle alk représente un groupement alkyle (C₁-C₆) linéaire ou ramifié, pour conduire à un ester de formule (VI) : 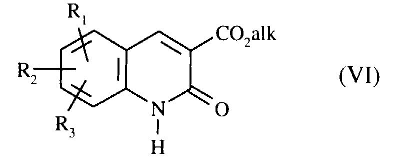 dans laquelle R₁, R₂, R₃ et alk ont la même signification que précédemment, que l'on transforme, en acide correspondant de formule (VII) : 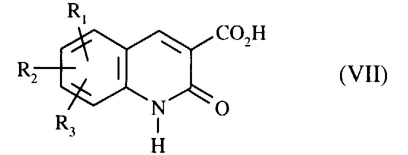 dans laquelle R₁, R₂ et R₃ ont la même signification que dans la formule (I),
que l'on met en réaction en présence de brome dans de la pyridine, pour conduire au composé de formule (VIII) : 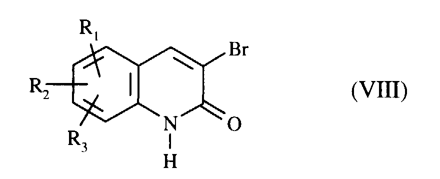 dans laquelle R₁, R₂ et R₃ ont la même signification que dans la formule (I),
que l'on fait réagir avec un composé de formule (IX) :
R'SH (IX)
dans laquelle R' représente un groupement alkyle (C₁-C₆) linéaire ou ramifié ou -CH₂CO₂alk (dans lequel alk a la même signification que précédemment),
pour conduire au composé de formule (X) : 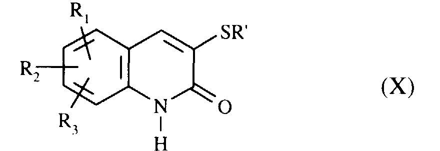 dans lequel R₁, R₂, R₃ et R' ont la même signification que dans la formule (I),
qui subit une oxydation en présence d'acide nitrique dans l'acide sulfurique concentré, pour conduire à l'acide sulfonique de formule (I) qui peut être éventuellement transformé en ester correspondant,
* soit condensé, avec un composé de formule (XI), en milieu protique, en présence d'un alcoolate de métal alcalin, d'une amine tertiaire ou d'un hydroxyde de métal alcalin : 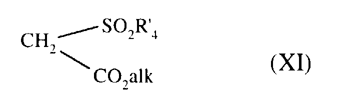 dans laquelle R'4 représente un groupement alkoxy (C₁-C₆) linéaire ou ramifié ou phénoxy éventuellement substitué, que l'on transforme :
après traitement éventuel en milieu acide, en composé de formule (I/a), cas particulier des composés de formule (I) : 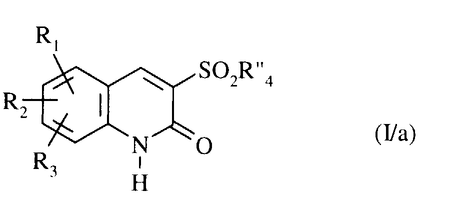 dans laquelle R₁, R₂ et R₃ sont tels que définis précédemment et R"₄ représente un groupement hydroxy, alkoxy (C₁-C₆) linéaire ou ramifié ou phénoxy éventuellement substitué,
composé de formule (I/a), que l'on transforme éventuellement lorsque R"₄ représente un groupement hydroxy, en chlorure du sulfonyle correspondant, puis par action de l'amoniac ou d'une amine, en composé de formule (I/b), cas particulier des composés de formule (I) : 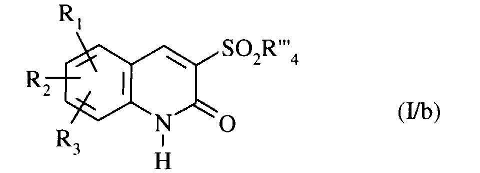 dans laquelle R₁, R₂, R₃ ont la même signification que dans la formule (I) et R'''₄ représente un groupement amino éventuellement substitué,
composé de formule (I/a) ou (I/b),
- qui, lorsque R₁ et/ou R₂ et/ou R₃ représentent un atome d'hydrogène, peut subir des substitutions électrophiles, selon des techniques classiques de substitution des noyaux aromatiques conduisant à un composé de formule (I) mono, di ou trisubstitué sur le noyau benzène de la quinoléinone,
- qui, lorsque R₁ et/ou R₂ et/ou R₃ représentent un groupement nitro, peut subir une hydrogénation pour conduire au dérivé amino correspondant, qui, lui-même, peut être, si on le souhaite, transformé en dérivé cyano correspondant,
- qui peut être, le cas échéant, purifié selon une technique classique de purification,
- dont on sépare, le cas échéant, les isomères selon une technique classique de séparation,
- que l'on transforme, si on le souhaite, en ses sels d'addition à un acide ou à une base pharmaceutiquement acceptable.

8. Compositions pharmaceutiques contenant comme principe actif au moins un composé selon l'une quelconque des revendications 1 à 6, seul ou en combinaison avec un ou plusieurs véhicules inertes, non toxiques, pharmaceutiquement acceptables.

9. Compositions pharmaceutiques selon la revendication 8 contenant au moins un principe actif selon l'une des revendications 1 à 6 utiles en tant qu'inhibiteurs des phénomènes pathologiques liés à l'hyperactivation des voies de neurotransmission aux aminoacides excitateurs, dans le traitement de l'accident vasculaire cérabral, du traumatisme cérébral ou spinal, de l'épilepsie et des maladies neurodégénératives chroniques telles que la sclérose amyotrophique latérale, la chorée de Huntington, la schizophrénie et la maladie d'Alzheimer.
